# EUROPEAN PATENT APPLICATION

(11) **EP 4 509 522 A1**
(43) Date of publication of application: **19.02.2025**
(21) Application number: 23191753.5
(22) Date of filing: 16.08.2023
(51) Int. Cl.: C07K 14/705, A61K 39/00, C07K 14/71, C07K 16/22

(54) **A TGFBETA SWITCH RECEPTOR, A NUCLEIC ACID ENCODING IT, CELLS AND PHARMACEUTICAL COMPOSITIONS COMPRISING THE SAME**

(71) Applicant: Max-Delbrück-Centrum für Molekulare Medizin in der Helmholtz-Gemeinschaft, 13125 Berlin (DE)
(72) Inventor: LORENZ, Felix, 13129 Berlin (DE); CLAUß, Julian, 13086 Berlin (DE); EDES, Inan, 13127 Berlin (DE); STAHN, Rainer, 13125 Berlin (DE); SADA JAPP, Alberto, 10439 Berlin (DE)
(74) Representative: Moré, Solveig Helga

(57) **Abstract**

The present invention relates to the field of adoptive immune cell therapy, in particular adoptive T cell therapy. The invention provides a switch receptor capable of efficiently converting the immunosuppressive signal of TGFβ that is typically present in the hostile tumor environment of solid tumors into a co-stimulatory signal that improves T cell activation and that is safe to use in therapy. Also provided are nucleic acids encoding the switch receptor, cell expressing said receptor and pharmaceutical compositions comprising said cells, in particular, for treatment of cancer or an infectious disease, e.g., by adoptive T cell therapy of a solid tumor.

## Description

The present invention relates to the field of adoptive immune cell therapy, in particular, adoptive T cell therapy. The invention provides a switch receptor capable of efficiently converting the immunosuppressive signal of TGFβ that is typically present in the hostile tumor environment of solid tumors into a co-stimulatory signal that improves T cell activation and that is safe to use in therapy. Also provided are nucleic acids encoding the switch receptor, cells expressing said receptor and pharmaceutical compositions comprising said cells, in particular, for treatment of cancer or an infectious disease, e.g., by adoptive T cell therapy of a solid tumor.

The tumor microenvironment is a hostile site in which T cells can be rendered dysfunctional by multiple metabolic and immunosuppressive mechanisms (1, 2). Transforming growth factor-β (TGFβ/ TGFb) is a pleiotropic cytokine frequently found in the tumor microenvironment of solid tumors where it acts as a potent immunosuppressant (3). It is produced by cancer-associated fibroblasts and by tumor cells themselves. TGFβ has multiple effects on T cells, resulting in reduced functionality such as inhibition of proliferation, exhaustion and suppression of cytolytic molecules and transcription factors (IFNy, granzyme B, perforin, Tbet and Eomesodermin) (3). Together, these effects lead to dysfunctional T cells and may result in poor anti-tumor responses. In fact, the presence of TGFβ is associated with disease progression (*4-6*) and failure of immune checkpoint inhibitor therapies (*7, 8*). Next-generation cellular immunotherapies for solid tumors need to take this hostile factor into account in order to ensure the functionality of the T cell product after infusion.

Different approaches to this issue have been taken in the state of the art. For example, Bendle GM, et al. (9) have used a dominant negative TGFβ receptor construct based on TGFβRII lacking the intracellular signaling domains for TGFβ signaling, and thus blocking its inhibitory signaling. In a clinical trial, four out of eight patients show an objective clinical response to adoptive T cell therapy with EBV-specific T cells expressing said construct (10).

In contrast to this blockade, other groups used a signal transformation receptor or switch receptor converting the immunosuppressive TGFβ signal into an immunostimulatory signal for the cell. For this, the extracellular domain of TGFβRII was fused with the intracellular signaling domain of CD137 (4-1BB). This improved the production of effector cytokines in T cells, and led to improved tumor eradication in an in vivo model of solid tumors (*11-13*). Similarly, WO2021244486 A1 (*14*), teaches a fusion protein comprising an extracellular portion of a receptor for an immunosuppressive cytokine, such as TGFβ and the intracellular region of one of a variety of co-stimulatory molecules.

Further, a TGFβ responsive polypeptide with an scFv binding domain derived from the PET1074B9 antibody and intracellular domains of CD28 and CD3ζ was used (*15*, *16*) . As the signal via this receptor alone is suitable for activating a T cell, the receptor can be considered a chimeric antigen receptor (CAR). It is therefore questionable if it is safe to use T cells for adoptive T cell transfer that express this switch receptor, as TGFβ can also be encountered in healthy tissue outside the context of the tumor.

In light of the state of the art, the inventors addressed the problem of providing an advantageous switch receptor that is capable of efficiently converting the immunosuppressive signal of TGFβ into a co-stimulatory signal that improves T cell activation through their TCR and that is safe to use in therapy of humans.

This problem is solved by the present invention, in particular, by the claimed subject-matter. The invention provides a nucleic acid encoding a switch receptor comprising the following domains
a) a TGFβ-binding domain that is an scFv,
b) a hinge domain,
c) a transmembrane domain,
d) an intracellular costimulatory signaling domain, wherein, after binding of the switch receptor to TGFβ, the intracellular costimulatory signaling domain is not capable of activating a T cell in the absence of further stimulation.

The switch receptor encoded by the nucleic acid is a continuous single chain polypeptide. Standard in the art, the domains are recited in the order from N-terminus to C-terminus of the polypeptide. Upon expression in a T cell expressing a T cell receptor (TCR) and, typically CD28, upon activation of the TCR and in the presence of TGFβ, the switch receptor of the invention is capable of increasing effector cytokine secretion, e.g., IFNy secretion, of said T cell compared to activation of the TCR in the absence of TGFβ.

The inventors generated novel switch receptors of the invention (also designated SWITCHes) and compared them with three state of the art receptors that bind to TGFβ and either provide a positive signal or no signal at all to the T cell. These receptors all have different structures and domains than the SWITCHes. The SWITCHes were benchmarked compared to the prior art receptors BM (benchmarking receptor) 1, BM2 and BM3. BM1 is the chimeric antigen receptor (CAR) against soluble TGFβ with an scFv binding domain derived from the PET1074B9 antibody and intracellular domains of CD28 and CD3ζ (*16*). BM2 is composed of the ectodomain of TGFβ receptor II (TGFβRII) and transmembrane and intracellular domains of CD137 (*12*). BM3 is a truncated version of TGFβRII, which binds soluble TGFβ but does not transmit any signal (*17*).

The structures of preferred SWITCH receptors of the invention are shown in Table 1.

**Table 1. 15 SWITCH (SR) constructs. The individual composition of each receptor is shown in the respective columns.**

| **Name** | **SR1-2** | **01** | **02** | **03** | **04** | **SR2-2** | **05** | **06** | **07** | **08** |
|---|---|---|---|---|---|---|---|---|---|---|
| **Binding domain** | PET1073G12 | | | | | PET1074B9 | | | | |
| **Leader** | Murine kappa light | | | | | | | | | |
| **Hinge** | IgG4 | IgG4 | IgG4 | CD8 | IgG4 | IgG4 | IgG4 | IgG4 | CD8 | IgG4 |
| **TM domain** | CD137 | CD8 | CD8 | CD8 | CD28 | CD137 | CD8 | CD8 | CD8 | CD28 |
| **IC domain** | CD137 | CD137 | CD8 + CD137 | CD8 + CD137 | CD137 | CD137 | CD137 | CD8+ CD137 | CD8+ CD137 | CD137 |

| **Name** | **SR3-2** | | **09** | | **10** | | **11** | | **12** | |
|---|---|---|---|---|---|---|---|---|---|---|
| **Binding domain** | PET1287A10 | | | | | | | | | |
| **Leader** | Murine kappa light | | | | | | | | | |
| **Hinge** | IgG4 | | IgG4 | | IgG4 | | CD8 | | IgG4 | |
| **TM domain** | CD137 | | CD8 | | CD8 | | CD8 | | CD28 | |
| **IC domain** | CD137 | | CD137 | | CD8 + CD137 | | CD8 + CD137 | | CD137 | |

The inventors found that all SWITCHes have an improved co-stimulatory effect over the BM that can be safely used in T cells for therapy of humans, and cells transduced with the switches of the invention have a higher IFNy secretion in the presence of TGFβ.

As explained in more detail below, BM1 and BM2 receptors were able to protect the T cells from the negative effect of TGFβ, leading to fold-changes of IFNy secretion in the presence of TGFβ of 3.4 and 1.2, respectively. BM3 cells did not fully circumvent TGFβ with a fold-change of 0.87. The SWITCH receptors of the invention all showed a fold-change above 1, i.e., they converted the negative signal into a positive one and led to increased IFNy secretion. All SWITCHes comprise a TGFβ - binding domain that is an scFv.

Of the prior art receptors of interest, the best immunostimulatory effect of TGFβ was seen with BM1, i.e., the only prior art receptor also comprising a TGFβ - binding domain that is an scFv. It however differs from the switch receptors of the present invention, in particular, by comprising intracellular domains of CD28 and CD3ζ, whereas the present receptors comprise a co-stimulatory domain of CD137. They do not comprise an intracellular signalling domain of CD3ζ and/or CD28.

All tested switch receptors of the invention outperformed the other state of the art benchmarking receptors except BM1. It is not surprising that BM1 provides a strong signal, as in contrast to the receptors of the invention, it comprises an intracellular signalling domain that is not even dependent on TCR stimulation. However, the switch receptors of the present invention further preferably comprise different scFv capable of binding TGFβ. SR01-04 outperformed the other constructs, including the benchmarking receptors, with a fold-change of IFNy secretion upon TCR stimulation in the presence of TGFβ between 3.5 and 5.

WO2006086469A2 compares the functional avidity of different anti-TGFβ scFv. Interestingly, PET1074B9 had the highest functional avidity, with PET1073G12 and PET1287A10 performing worse. However, the present experiments surprisingly show that said characteristic does not predict the most advantageous scFv for use in the SWITCH receptor of the present invention. The inventors could show that, contrary to expectation, in the context of a construct of the present invention, use of an scFv based on PET1073G12 led to the best results.

The SWITCH receptor of the invention preferably has a functional avidity to TGFβ of at most 50 ng/mL, e.g., in the range of 0.1 - 50 ng/mL, 0.4 - 10 ng/mL, 0,5 - 5 ng/mL. preferably, 0.7 - 2 ng/mL. The TGFβ can, in the context of the invention, be TGFβ1, TGFβ2 or TGFβ3, preferably, TGFβ1. Typically, the human system is of highest relevance, and the TGFβ is human.

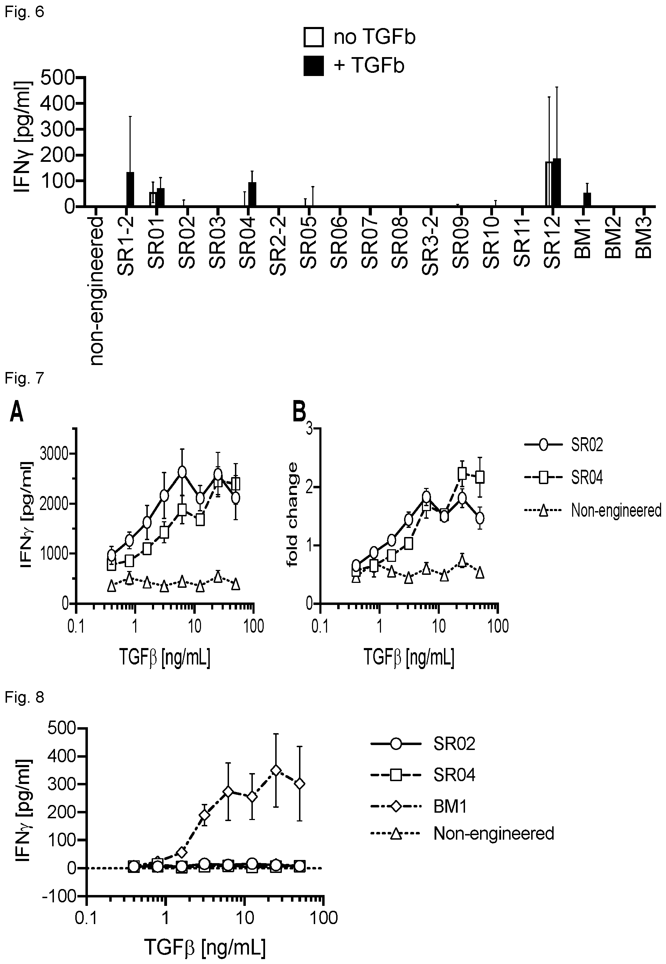

Functional avidity of the SWITCH as such can be measured by the increase in IFNy secretion when SWITCH-expressing T cells are activated by anti-CD3/anti-CD28 beads in the presence of TGFβ, e.g., as disclosed in MATERIALS & METHODS and Figure 7A.

In a preferred embodiment of the invention, the scFv comprises a variable heavy (VH) and a variable light (VL) domain, wherein
a) the VH domain comprises a CDR1 of SEQ ID NO: 1, a CDR2 of SEQ ID NO: 2 and a CDR3 of SEQ ID NO: 3 and the VL domain comprises a CDR1 of SEQ ID NO: 4, a CDR2 of SEQ ID NO: 5 and a CDR3 of SEQ ID NO: 6. An scFv on this basis, i.e., comprising these CDRs, is designated PET1073G12, and SWITCHes of the invention having this scFv were shown to be particularly advantageous. Suitable framework regions are e.g., disclosed in WO2006086469A2. They can be human or murine framework regions, preferably, human. PET1073G12 preferably comprises a variable heavy (VH) domain of SEQ ID NO: 7 and/or a variable light (VL) domain of SEQ ID NO: 8, preferably, both. The VH and VL domain can be in any order, but preferably the VH domain is N-terminal, as this led to better expression. The scFv may e.g. have a sequence of SEQ ID NO: 9, as in SWITCHes of Table 1. Said scFv is optionally encoded by SEQ ID NO: 10.

In an alternative embodiment of the invention, the scFv comprises a variable heavy (VH) and a variable light (VL) domain, wherein
b) the VH domain comprises a CDR1 of SEQ ID NO: 11, a CDR2 of SEQ ID NO: 12 and a CDR3 of SEQ ID NO: 13 and the VL domain comprises a CDR1 of SEQ ID NO: 14, a CDR2 of SEQ ID NO: 15 and a CDR3 of SEQ ID NO: 16. An scFv on this basis is designated PET1074B9. Suitable framework regions are e.g., disclosed in WO2006086469A2. They can be human or murine framework regions, preferably, human. PET1074B9 preferably comprises a VH domain of SEQ ID NO: 17 and/or a VL domain of SEQ ID NO: 18, preferably both. The VH and VL domain can be in any order, but preferably the VH domain is N-terminal, as this led to better expression. The scFv may e.g. have a sequence of SEQ ID NO: 19, as in SWITCHes of Table 1. Said scFv is optionally encoded by SEQ ID NO: 20.

In an alternative embodiment of the invention, the scFv comprises a variable heavy (VH) and a variable light (VL) domain, wherein
c) the VH domain comprises a CDR1 of SEQ ID NO: 21, a CDR2 of SEQ ID NO: 22 and a CDR3 of SEQ ID NO: 23 and the VL domain comprises a CDR1 of SEQ ID NO: 24, a CDR2 of SEQ ID NO: 25 and a CDR3 of SEQ ID NO: 26. An scFv on this basis is designated PET1287A10. Suitable framework regions are e.g., disclosed in WO2006086469A2. They can be human or murine framework regions, preferably, human. PET1287A10 preferably comprises a VH domain of SEQ ID NO: 27 and/or a VL domain of SEQ ID NO: 28, preferably, both. The VH and VL domain can be in any order, but preferably the VH domain is N-terminal, as this led to better expression. The scFv may e.g. have a sequence of SEQ ID NO: 29, as in SWITCHes of Table 1. Said scFv is optionally encoded by SEQ ID NO: 30.

The scFv comprised in the SWITCH of the invention may e.g. comprise PET1073G12 of SEQ ID NO: 9, b) PET1074B9 of SEQ ID NO: 19 and c) PET1287A10 of SEQ ID NO: 29, or a variant of any of said scFv having at least 90% sequence identity to any of SEQ ID NO: 9, 19 or 29. Of course, said variant comprises the CDR regions recited herein and specifically binds TGFβ.

Generally, the different functional parts or domains of the switch receptor of the invention can e.g., be derived from human, mouse, rat, dog, donkey, goat, or rabbit. Preferably, in particular, if intended for use in treatment of a human subject, at least the hinge, transmembrane and intracellular domains are human, optionally, all domains. It is also preferred that the framework regions of the scFv are human. Use of human sequences is advantageous to minimize immunogenicity in humans. Similarly, in context with treatment of a murine/mouse subject or in a mouse cell or mouse experiment, optionally, these domains are preferably murine.

In the switch receptor of the invention, there is a hinge domain C-terminal to the scFv. Said hinge domain can also be considered a spacer and allows for better accessibility of the scFv to TGFβ. It may a have a length of 3-100 aa, 4-50 aa, 5-30 aa, 6-25 aa, 7-20 aa, 8-15 aa, 9-14 aa, 10-13 aa or 11-12 aa. The hinge domain may be any suitable hinge region, e.g., as known in the art in the context of CARs. However, as CARs often bind to cell-surface antigens, typically, "long" hinges, e.g., having 30 aa or more are typically chosen for them. Such hinges can be used, e.g., an extracellular portion of CD8a as taught by EP3769816A1 (18) (Protein ID NO: AAB04637.1). In the present case, however, shorter hinges are sufficient, as the ligand is a soluble cytokine. Thus, the hinge can preferably have about 7-15 aa, e.g., 10-13 aa, such as 12 aa.

In some embodiments, the hinge is the hinge region of an IgG molecule. For example, it may comprise a CH2CH3 region of an IgG molecule. In some embodiments, the peptide spacer comprises one or more of a hinge region, CH1, CH2, and CH3 region. In some embodiments, the peptide spacer is derived from a hinge, CH1, CH2, and/or CH3 region or other region (preferably, a hinge region) of an IgG1, IgG2, IgG3, IgG4, IgA1, IgA2, IgD, IgE, or IgM from human, mouse, rat, dog, donkey, goat, or rabbit (preferably, human). In some embodiments, the peptide spacer comprises the hinge region of an IgG molecule. If it further comprises the CH2CH3 region of an IgG molecule, it may have additional L235E/N297Q or L235D/N297Q mutations to prevent Fc receptor binding. In some embodiments, the hinge consists of the hinge region of an IgG molecule. In a preferred embodiment, the hinge region may comprise or consist of a human IgG4 hinge domain, e.g., of (SEQ ID NO: 31). This is, e.g., the case in preferred SWITCHes of the invention, SR02 and SR04. SR02 differs only in the hinge region from SR03, and has a reproducibly stronger co-stimulatory effect. Therefore, the hinge domain preferably is an IgG4 hinge domain.

Other SWITCHes of the invention have a CD8 hinge domain, e.g., CD8a or CD8β, preferably, human CD8a. It may comprise the sequence of SEQ ID NO: 32 or consist thereof. As the adjacent transmembrane domain can also derive from CD8, use of this combination, e.g., as in SR03, can simplify cloning and reduce the risk of formation of potentially immunostimulatory epitopes.

C-terminal to the hinge domain, the transmembrane domain anchors the switch receptor in the cell membrane. It allows for transductions of the signal from the extracellular to the intracellular part of the molecule. It is interposed between the hinge domain and the intracellular domain, and it can be directly adjacent to those. Use of further linkers is possible, but not required. Preferably, the transmembrane domain is derived from a co-stimulatory molecule. For example, the transmembrane domain may be selected from the group comprising transmembrane domains of CD8 (CD8a or CD8β, preferably, CD8a) (e.g., of SEQ ID NO: 33), CD28 (e.g., of SEQ ID NO: 34) or CD137 (e.g., of SEQ ID NO: 35). It can also be a transmembrane domain as described in WO2014172584A1, e.g., of CD134 or CD7. Transmembrane domains allowing for transduction of the TGFβ signal from the extracellular to the intracellular part of the molecule having variants of the specific sequences recited herein can be used, e.g., having at least 90% sequence identity to any of SEQ ID NO: 33-34. A CD137 transmembrane domain appeared to negatively affect expression, and is thus generally less preferred. SWITCHes with CD8 or CD28 transmembrane domains were shown to have excellent expression.

Interestingly, the inventors could show that use of the transmembrane domain of CD8, in particular, CD8a was most advantageous in the switch receptor of the invention. For example, SR02 performed still better than SR1-2 and SR04, which have transmembrane domains derived from CD137 and CD28, respectively.

In the intracellular part, all switch receptors of the invention comprise an intracellular signaling domain that is a co-stimulatory signaling domain. Thus, the intracellular signaling domain is capable of providing a co-stimulatory T cell signal, i.e., the signal can, together with a signal of an activated TCR, activate a T cell. It can however not activate a T cell in the absence of further stimulation, in particular, in the absence of stimulation through a TCR or CAR stimulation. It thus does not comprise the intracellular domain of a CD3 chain, in particular CD3ζ.

The intracellular signaling domain preferably is an intracellular domain of an immunoglobulin superfamily or TNF receptor superfamily protein, e.g., as disclosed in (12). The intracellular domain can be e.g., an intracellular signaling domain of CD137 (4-1BB), CD134 (OX40), CD27, TNFRSF18 (GITR or AITR), CD270 (HVEM or TNFRSF14), CD154 (CD40L), CD28, CD278 (ICOS), CD226 (DNAM-1), CD4 or CD8. An intracellular domain of a T cell stimulatory cytokine receptor such as IL-2, IL-7, IL-12 or IL-15 may also be used. It may also be the intracellular domain of TIM (T cell immunoglobulin mucin domain, e.g., TIM-1), CD2/SLAM or a butyrophilin such as a BTN or a BTN-like. It may also be a combination of such signaling domains. In a preferred embodiment, the intracellular signaling domain comprises at least one, e.g., one or two intracellular signaling domain of CD137, CD28, CD4 and/or CD8. It may optionally comprise at least one immunoreceptor tyrosine-based activation motif (ITAM) and/or be able to bind TRAF (TNF receptor associated factor). Preferably, the switch receptor of the invention comprises one intracellular signaling domain, optionally, in combination with a spacer (e.g., a part of CD4 or CD8 (preferably, CD8a) intracellular domains).

Preferably, the intracellular signaling domain is the intracellular signaling domain of CD137 or a combination of an intracellular part of human CD8 and the intracellular signaling domain of human CD137. It can also be the intracellular signaling domain of CD28 or a combination of an intracellular part of human CD8 and the intracellular signaling domain of human CD28.

In a preferred embodiment, the switch receptors of the invention comprise the intracellular signaling domain of CD137, preferably, human CD137. Preferably, the switch receptor of the invention comprises the intracellular signaling domain of SEQ ID NO: 36. The domain can also be a variant thereof having at least 90% sequence identity that is capable of initiating CD137-mediated signaling. CD137 is a member of the TNF-receptor (TNFR) superfamily without known intrinsic enzymatic activity in its intracellular domain. It relies on the TNFR-Associated-Factor (TRAF) family of adaptor proteins to build the CD137 signalosome for transducing signals into the cell. Thus, upon binding of the ligand, in the present case, upon binding of TGFβ to the scFv part of the SWITCH, TRAF1, TRAF2, and TRAF3 are recruited to the intracellular signaling domain of CD137, likely as homo- and/or heterotrimers with different configurations, initiating the construction of the CD137 signalosome. The signaling domain can thus recruit TRAF1, TRAF2, and TRAF3 upon binding of TGFβ to the scFv part of the SWITCH.

It can be beneficial to include a spacer between the transmembrane domain and intracellular signaling domain, e.g., the CD137 intracellular signaling domain. In particular, the switch receptor may comprise a combination of an intracellular part of CD8, preferably, CD8α, and the intracellular signaling domain, e.g., of human CD137. Combinations of an intracellular part of CD8 and CD137 disclosed in EP3769816A1 in a different context can be used. The intracellular part of CD8a can be human and may have, e.g., SEQ ID NO: 37. The intracellular part of CD8 can also be a variant of SEQ ID NO: 37 having at least 90% sequence identity thereto, wherein the combination of said intracellular part of CD8 and the intracellular signaling domain of human CD137 is capable of initiating CD137-mediated signaling. An intracellular part of CD8β may alternatively be used.

The data shown below demonstrates that inclusion of the intracellular part of CD8 is advantageous in context of SR02 (as compared to SR01), as well as the SWITCHES based on other scFv. This is particular preferred if the transmembrane domain is also derived from CD8. Interestingly, however, the costimulatory effect does not appear to be better if, further, the hinge domain is also derived from CD8.

Thus, preferred SWITCHES of the invention comprise a hinge domain derived from IgG4, a transmembrane domain derived from CD8 and a combination of an intracellular part of CD8 and the intracellular signaling domain of human CD137. This appears to be particularly advantageous in the context of a switch receptor comprising PET1073G12. Interestingly, for constructs comprising the other tested scFv, SR08 and SR12 proved to be more effective in converting the TGFβ signal to a costimulatory signal than SR06 and SR10, respectively. Of note, SR04 also shows excellent functions. Thus, another preferred SWITCH comprises a hinge derived from IgG4, a transmembrane domain derived from CD28 and the intracellular signaling domain of human CD137. While the intracellular part can comprise a combination with an intracellular part of CD8, as disclosed herein, preferably, said construct does not comprise a combination with an intracellular part of CD8.

Further, the inventors showed that use of both a CD137 transmembrane domain and an intracellular signaling domain of CD137 (without a CD8 part in between) led to a diminished expression of the construct compared to other switch receptors of the invention.

To simplify detection of expression of a receptor of the invention on a cell, the switch receptor may further comprise an extracellular tag, optionally, a FLAG tag (SEQ ID NO: 38), c-myc-Tag, CD34-tag, His-tag, or a HA-tag, preferably, a FLAG tag. Said tag can be located N-terminal to the TGFβ - binding domain. It can optionally be flanked by a linker, e.g., a glycine serine linker such as GGS, GSS or GSG linker, between the tag and the TGFβ - binding domain. In principle, every tag to which a ligand or, preferably, an antibody is available can be used. Preferably, the tag is not highly immunogenic or not immunogenic in a human subject, which is most important in settings of therapy in human subject. There are normally no pre-existing antibodies, though, and a single administration may suffice. In an alternative embodiment, however, the receptor of the invention does not comprise an additional extracellular tag (or detectable marker). This is not required because the TGFβ - binding domain and/or the hinge domain can be used for detecting expression. For example, the TGFβ - binding domain can be detected with labelled TGFβ. Antibodies to the receptor, e.g., to framework regions of the scFv or to the hinge region can also be used. Thus, the risk of an immune response against transferred T cells carrying the switch receptor of the invention can be minimized.

To enable transmembrane expression of the switch receptor of the invention, it typically comprises an N-terminal signal peptide, also designated a leader or leader sequence herein. Said sequence can be, e.g., a signal peptide for expression of a transmembrane protein, such as an antibody chain, e.g., of a murine kappa light chain, murine lambda light chain, murine heavy chain, human kappa light chain, human lambda light chain, human heavy chain, human HLA, human TCR alpha, human TCR beta, human TCR gamma, or human TCR delta. Interestingly, expression was improved with a murine kappa light chain leader sequence compared to the human kappa light chain leader sequence or human heavy chain leader sequence. Thus, preferably, the switch receptor further comprises an N-terminal murine kappa light chain leader sequence (e.g., SEQ ID NO: 40). C-terminal to said leader sequence follows, optionally, a tag, and the scFv sequence. Spacers, e.g., glycine serine linkers, may further be present between leader and tag and/or tag and scFv and/or leader and scFv domains.

The switch receptor of the present invention may be any of SR01 to SR12 or SR1-2, SR2-2 or SR3-2. It may thus have the sequence of any of SEQ ID NO: 41-55 or at least 90%, e.g., at least 95% or at least 99% sequence identity to any of SEQ ID NO: 41-55. Preferably, the receptor has SEQ ID NO: 42 (SR02) or at least 90%, e.g., at least 95% or at least 99% sequence identity thereto. Most preferably, sequence identity is 100%.

In the nucleic acid of the invention encoding the switch receptor, sequences encoding the switch receptor of the invention are typically functionally linked to a promotor region, in particular, a promotor allowing for expression of the receptor in a T cell, preferably, a human T cell. The promotor can be constitutive or inducible. For example, the promotor can be human EF1a, PGK, beta actin, IF4A1, GAPDH, GRP78, HSP70, beta kinesin, ubiquitin B, CD45, MP71, or CMV. Preferably, the promotor is EF1a. The nucleic acid can thus be considered an expression construct for the switch receptor. The sequences encoding the switch receptor of the invention may be directly linked to the promotor. Alternatively, functional linkage to a promotor may be in the context of a polycistronic operon, wherein different encoded polypeptides are encoded under the control of one promotor, and separated, e.g., by an internal ribosomal entry side (IRES) or a 2A element. Such other polypeptides may e.g., encode the alpha and beta chains of a TCR. Optionally, a CD8 co-receptor may also be encoded.

2A elements may be, e.g., P2A, E2A, F2A, or T2A. Different 2A peptides have different efficiencies of self-cleaving, T2A and P2A being the most and F2A the least efficient. A P2A element is preferred in the context of the invention, e.g., in combination with a glycine serine linker, e.g., a GSG linker, directly N-terminal to the P2A element. Adding the optional linker "GSG" N-terminal of a 2A peptide helps with efficiency, and is therefore preferred for all P2A elements. There may also be a linker such as a GSG linker C-terminal of cleavage sites.

The nucleic acid of the invention may be a vector, such as an expression vector. It typically is DNA. The nucleic acid may e.g. be a minicircle, a plasmid, a nanoplasmid, doggybone DNA or RNA or modified RNA.

Recently DNA constructs using doggybone DNA (dbDNA), synthetic closed linear DNA, have been developed. The cell-free process relies on the use of amplification, e.g., with Phi29 DNA polymerase, wherein amplification of the template is followed by incubation with protelomerase TelN to complete individual closed linear DNA. The resulting dbDNA may e.g., contain the required sequences, a promoter and a poly A tail, but lacks bacterial sequences such as an antibiotic resistance gene.

A minicircle is derivable from a plasmid wherein bacterial sequences such as the origin of replication have been excised. Minicircles are small (typically smaller than 5 kb, preferably, about 4kb) circular plasmid derivatives that have been freed from all prokaryotic vector parts. Minicircles have been applied as transgene carriers for the genetic modification of mammalian cells, with the advantage that, since they contain no bacterial DNA sequences, they are less likely to be perceived as foreign and destroyed. The smaller size of minicircles also extends their cloning capacity and facilitates their delivery into cells, e.g., using transposases. Suitable nucleic acids and protocols are e.g., disclosed in WO 2017/158019 A1 (19).

Minicircles lack an origin of replication, so they typically do not replicate within the target cells and the encoded genes will disappear as the cell divides. A novel addition to the field are nonviral self-replicating minicircles, which owe this property to the presence of a S/MAR-Element. Nanoplasmids are small circular DNA constructs without antibiotic resistance genes and having a backbone smaller than 500 bp.

Plasmids (circular DNA comprising an origin of replication) can also be used in the context of the invention, but it is preferred that less DNA needs to be transferred, so preferably, the nucleic acid is a doggybone DNA, a nanoplasmid or a minicircle, most preferably, a minicircle.

In one embodiment, the DNA is a transposon or comprises a transposon. Consequently, the nucleic acid encoding the required sequences may be flanked by inverted repeats capable of being mobilized by a transposase, preferably, by a Sleeping Beauty transposase such as SB100X. Suitable transposases are e.g., disclosed in WO 2009/003671 A2 (20). Suitable transposons are e.g., disclosed in WO 2017/158029 A1 (21). For example, the vector may be a p2 vector, or, preferably, a p4 or p5 vector.

The vector may also be a viral vector, e.g., a retroviral, adenoviral, AAV, or lentiviral vector.

The invention also provides a switch receptor encoded by the nucleic acid of the invention. The switch receptor may be an isolated protein, or it may be expressed as a cell surface molecule of a cell.

The invention also discloses a method of preparing a cell comprising the nucleic acid of the invention. The vector, e.g., the purified minicircle can be transferred into the recipient cell by lipofection, jet injection, squeezing, nanoneedles, or other transfection methods, or, preferably, electroporation. Infection of viral vectors is another preferred method of preparing a cell of the invention. RNA, for example, can easily be transferred by electroporation.

The present invention also provides a cell comprising the nucleic acid of the invention, wherein the cell preferably also expresses the switch receptor of the invention. The cell can be a bacterial cell, e.g., E. coli, but it is preferably an eukaryotic cell, e.g., a mammalian cell. Typically, for research purposes, the cell is a mouse or human cell. Therapeutic cells are usually human cells, and a human subject is to be treated. The cell preferably is a T cell, NK cell or NKT cell, most preferably, a T cell. T cells can be alpha beta T cells, in particular they may comprise CD8 alpha beta T cells or consist thereof. More preferably, T cells are a mixture of CD4 and CD8 T cells. T cells can also be gamma delta T cells.

The cell of the present invention preferably further expresses a class-I-restricted TCR construct. Said TCR may be directed to a tumor antigen, e.g., a tumor antigen expressed by a solid tumor, such as a MAGE antigen (e.g., MAGEA1, MAGEA2, MAGEA3, or MAGEA4). The TCR may also be directed, e.g., to an EBV antigen or another tumor antigen.

For treatment, T cells, e.g., alpha beta or gamma delta T cells engineered with the nucleic acid of the invention can be allogeneic, to allow for off-the shelf solutions, but they are typically syngeneic, e.g., autologous. This avoids immune rejection or the need for immune suppression to avoid graft versus host disease (GvHD).

The invention also provides a pharmaceutical composition comprising the nucleic acid of the invention, the switch receptor of the invention or the cell of the invention. In particular, the pharmaceutical composition may comprise a cell of the invention that is a T cell, NK cell or NKT cell. The pharmaceutical composition optionally further comprises a suitable buffer and/or excipient.

The pharmaceutical compositions of the invention typically are for intravenous administration. They may comprise pharmaceutically acceptable carriers such as buffers, e.g., physiological saline or PBS. They may comprise excipients, e.g., stabilizers such as SPGA, carbohydrates (e.g. sorbitol, mannitol, starch, sucrose, glucose, dextran), proteins such as albumin or casein, or protein containing agents such as bovine serum or skimmed milk. If the composition does not comprise cells but nucleic acids, it can be a dry composition, e.g., a lyophilized composition. Such compositions typically comprise a bulking agent, e.g., a non-reducing sugar such as trehalose. They may also comprise a buffering agent.

T cells are typically administered to a subject or patient in a concentration of 1 × 10⁵ - 5 × 10⁹ cells per kg bodyweight. About 1 × 10⁶ - 5 × 10¹¹ cells, e.g., 1 × 10⁸ - 2 × 10¹⁰ cells may be administered to a human subject as a single dose, i.v.. These parameters may be adapted by the medical practitioner, depending, e.g., on the patients age, sex, body weight and medical condition.

The pharmaceutical composition of the invention in one embodiment comprises autologous T cells of the subject which were in vitro-engineered to express a nucleic acid of the present invention, e.g., autologous CD4 and/or CD8 T cells.

Alternatively, the subject may also be administered a nucleic acid of the invention, in particularly, an expression vector, for in vivo engineering, i.e., in vivo transduction of T cells. The expression vector can e.g. be targeted to T cells via a specific antibody recognizing surface molecule(s) expressed exclusively by the cells to be engineered, e.g. CD3, CD4, CD8, TCR alpha, TCR beta, TCR gamma and/or TCR delta.

The present invention also provides the pharmaceutical composition of the invention for use in treatment of a subject in need thereof, in particular, a subject having a cancer (including those caused by infectious agents) or an infectious disease, preferably, a cancer such as a solid cancer. A subject that has a disease, e.g., a cancer, is also designated a patient herein. Preferably, the pharmaceutical composition is for use in adoptive T-cell therapy, e.g., adoptive T cell therapy of cancer.

It may be adoptive T cell therapy or T cell receptor (TCR) gene therapy, e.g., directed to an epitope derived from the cancer-testis antigen MAGEA4 presented on HLA-A*01, e.g., with the TCR CTC127 (as disclosed in WO 2022/243514 A1 (22)) or a variant thereof having at least 90 % sequence identity in the CDR1, CDR2 and CDR3 regions, and preferably, having at least 98% or at least 99% sequence identity to CTC127 over the complete sequence of the variable regions.

Also disclosed is a method of treating a subject in need thereof, e.g., a subject having a cancer, preferably, a solid cancer. The therapy typically comprises intravenous administration of the pharmaceutical compositions of the invention, preferably, infusion. Typically, before administration of T cells, the patients are pre-conditioned, e.g., by administration of fludarabine and cyclophosphamide (e.g., infusions of 30 mg/m² fludarabine and 500 mg/m² cyclophosphamide), for example, on days 5, 4 and 3 prior to administration of T cells.

The subject typically is a mammalian subject, e.g., a mouse or a human. Preferably, it is a human patient.

The solid cancer can be, e.g., melanoma, gastric cancer, head and neck, lung, breast, ovarian, cervical, bladder, synovial sarcoma, liposarcoma, and oesophageal cancer. TGFβ is expressed at high levels in all of these solid tumors. Cancers may also be caused by CMV, Kaposi sarcoma virus, *H. pylori,* Merkel cell polyomavirus, or papillomavirus (HPV). The infectious disease can be caused by an intracellular protozoan, e.g., *Trypanosoma* spp, *Leishmania* spp., *Toxoplasma* spp., or *Mycobacterium* spp., HCV, HIV, and parainfluenza virus. TGFβ expression plays a role in down-regulating immune responses in relevant infectious diseases.

The present invention is further illustrated in the following examples with reference to the accompanying figures and sequences, nevertheless, without being limited thereto. For the purposes of the present invention, all references as cited herein are incorporated by reference in their entirety.

### Legends

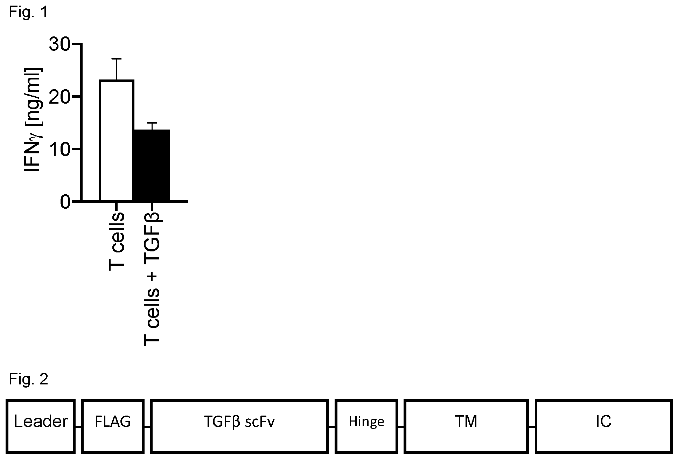

Fig. 1. IFNy release by T cells in the presence of TGFβ. T cells from 2 donors were expanded with a lab-scale protocol for 9 days prior to restimulation with aCD3/aCD28 beads (1:100 dilution) in the presence or absence of 10 ng/ml human recombinant TGFβ for 24 hours. IFNy in the supernatant was measured via ELISA. Graph shows mean±SEM IFNy concentration from replicates of 2 donors.
Fig. 2. Modular building blocks of switch receptors. Leader: murine kappa light. FLAG: tag for specific detection and/or selection of the SWITCH. TGFβ binding domain: VH and VL chains of one of three human anti-TGFβ antibodies. Hinge: human IgG4 or CD8. Transmembrane (TM) domain: human CD137, CD8, or CD28. Signaling domain: human CD137 or truncated human CD8 together with CD137.

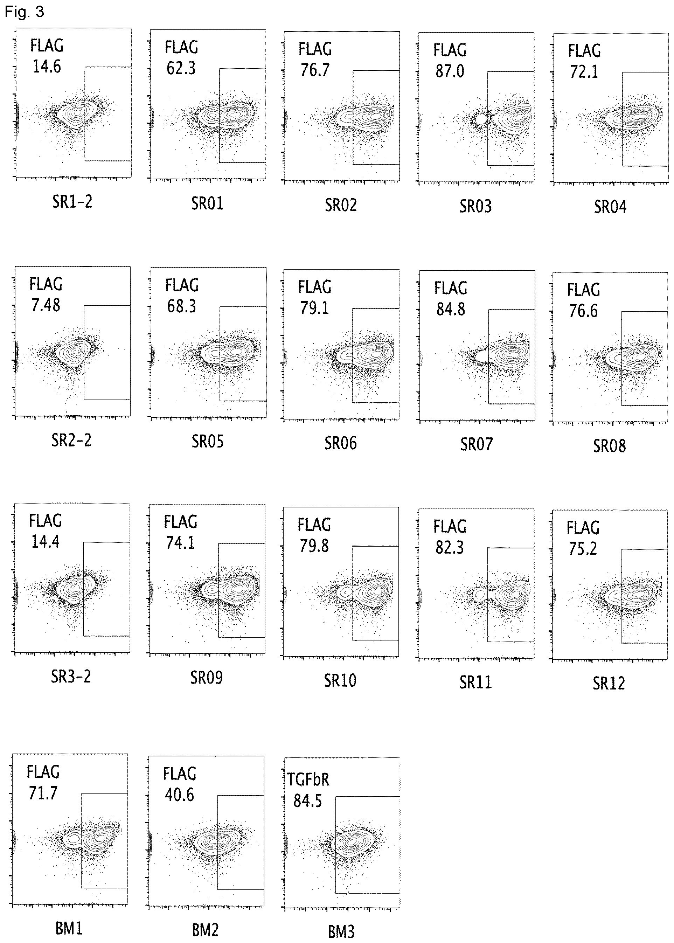

Fig. 3. Cell surface expression of SWITCH and BM receptors. Human CD8⁺ T cells were retrovirally transduced with the 15 SWITCH candidates or with 3 BM receptors. Transduction efficiency and surface expression levels were assessed on day 5 after transduction by flow cytometric staining of the FLAG tag or TGFβRII (only for BM3). Dot plots show live T cells from 1 of 2 donors. Number represents frequency of cells expressing the engineered receptors.

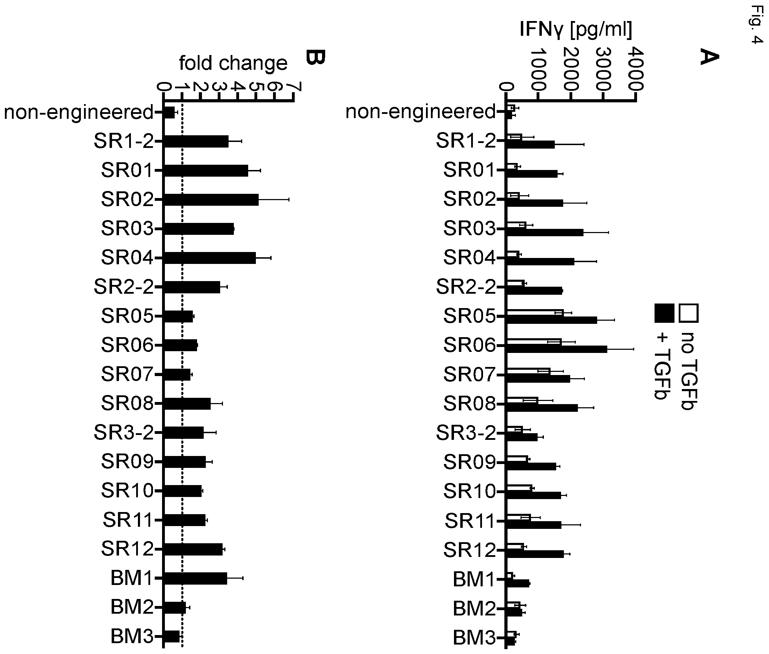

Fig. 4. Functional assessment of SWITCH candidates and benchmarking receptors. A total of 10⁴ T cells were stimulated with aCD3/aCD28 beads (1:100 dilution) for 24 hours in the presence or absence of 10 ng/ml recombinant human TGFβ. IFNy was detected in the supernatant (1:5 dilution) by ELISA. A) IFNy concentration for both experimental conditions (with and without TGFβ). B) Fold change in IFNy secretion in the presence of TGFβ. Dotted line marks a fold change of 1, i.e., no difference between the two conditions. Data show mean ± SEM of replicates of two T cell donors.

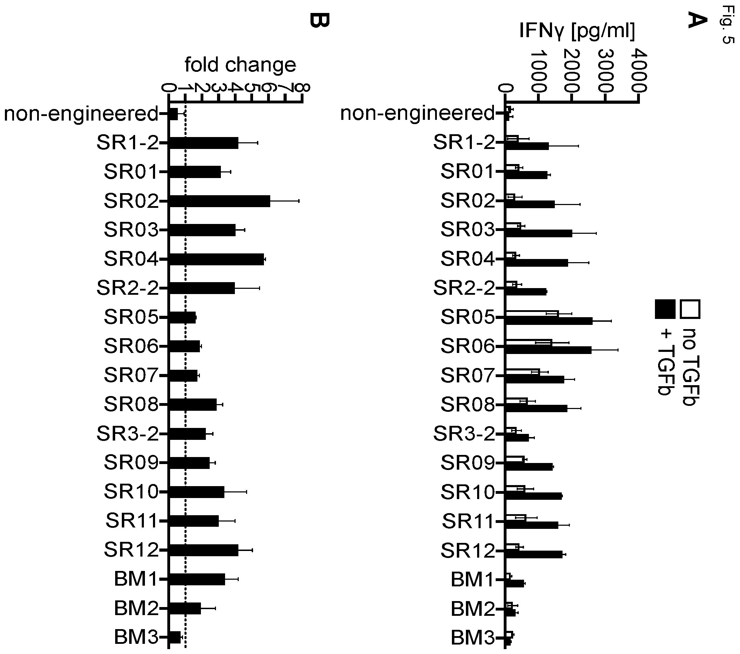

Fig. 5. Functional assessment of SWITCH candidates and benchmarking receptors. A total of 10⁴ T cells were stimulated with aCD3/aCD28 beads (1:300 dilution) for 24 hours in the presence or absence of 10 ng/ml recombinant human TGFβ. IFNy was detected in the supernatant (1:5 dilution) by ELISA. A) IFNy concentration for both experimental conditions (with and without TGFβ). B) Fold change in IFNy secretion in the presence of TGFβ. Dotted line marks a fold change of 1, i.e., no difference between the two conditions. Data show mean ± SEM of replicates of two T cell donors.
Fig. 6. Assessment of T cell activation by SWITCHs in the presence of TGFβ but absence of a TCR (CD3/CD28) stimulus. A total of 10⁴ T cells were cultivated for 24 hours in the presence or absence of 10 ng/ml recombinant human TGFβ. IFNy was detected in the supernatant (1:5 dilution) by ELISA. Data show mean ± SEM of replicates of two T cell donors.
Fig. 7 In-depth functional assessment of top SWITCH candidates. A total of 10⁴ T cells were stimulated with a suboptimal concentration of aCD3/aCD28 beads (1:300 dilution) for 24 hours in the presence or absence of human TGFβ in a range of concentrations (0, 0.4, 0.8, 1.6, 3.1, 6.2, 12.5, 25 and 50 ng/mL). IFNγ was detected in the supernatant (1:5 dilution) by ELISA. A) IFNy concentration (value for 0 TGFβ cannot be shown in the log scale) B) Fold change in IFNy secretion in the presence of TGFβ compared to secretion without TGFβ. Data show mean ± SEM of replicates of two T cell donors.
Fig. 8 In-depth functional assessment of top SWITCH candidates and benchmarking receptor 1 (BM1) in the absence of a TCR stimulus. A total of 10⁴ T cells were cultured for 24 hours in the presence of human TGFβ in a range of concentrations (0.4, 0.8, 1.6, 3.1, 6.2, 12.5, 25 and 50 ng/mL). IFNy was detected in the supernatant by ELISA. Data show mean ± SEM of replicates of two T cell donors.

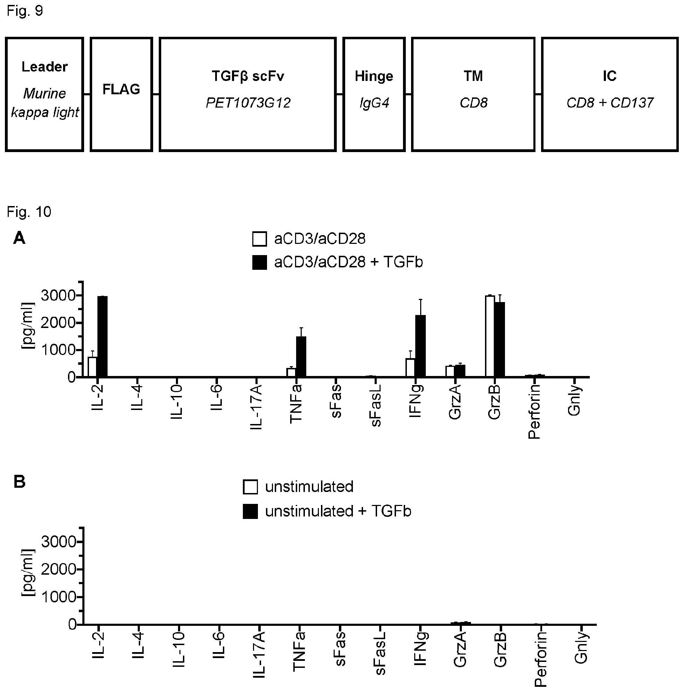

Fig. 9. Modular structure of lead candidate: SR02.
Fig. 10. Assessment of polyfunctionality. A total of 10⁴ SR02⁺ T cells were stimulated with aCD3/aCD28 beads (1:300 dilution) (A) or left unstimulated (B) for 24 hours in the presence or absence of 10 ng/ml recombinant human TGFβ. Cytokines were detected in the supernatant using a cytokine bead array (CBA) from BioLegend according to their instructions.

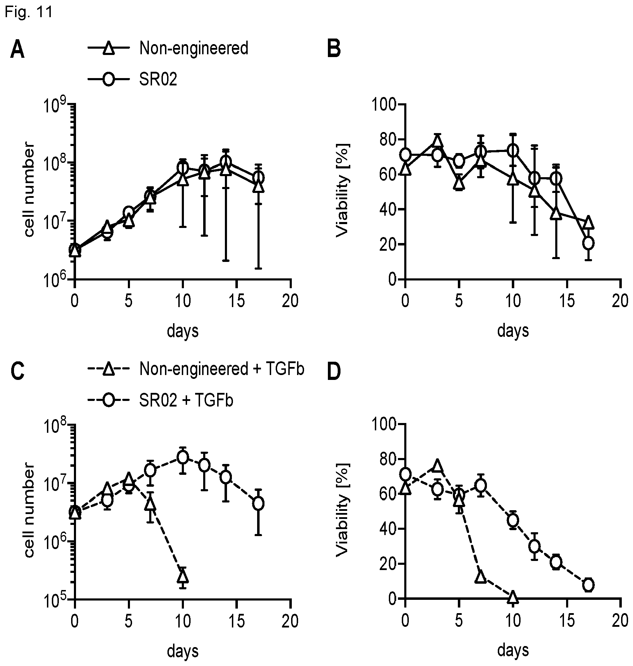

Fig. 11. Assessment of cell proliferation and survival upon repeated stimulations with TGFβ. Non-engineered and SR02+ T cells were repeatedly stimulated with aCD3/aCD28 beads (1:300 dilution) in the presence or absence of TGFβ. A total of 7 stimulations were performed over the course of 17 days (at days 0, 3, 5, 7, 10, 12, and 14). Between each round of stimulation, cell count and viability were assessed and cells were adjusted to 8×10⁵ cells/mL for the next stimulation round A) Cell numbers in the absence of TGFβ. B) Cell viability in the absence of TGFβ. C) Cell numbers in the presence of TGFβ. D) Cell viability in the presence of TGFβ.

### Sequences

SEQ ID NO: 1 HCDR1 / PET1073G12
SEQ ID NO: 2 HCDR2 / PET1073G12
SEQ ID NO: 3 HCDR3 / PET1073G12
SEQ ID NO: 4 VCDR1 / PET1073G12
SEQ ID NO: 5 VCDR2 / PET1073G12
SEQ ID NO: 6 VCDR3/ PET1073G12
SEQ ID NO: 7 Heavy chain variable domain / PET1073G12
SEQ ID NO: 8 Light chain variable domain / PET1073G12
SEQ ID NO: 9 scFv / PET1073G12 including (G4S)3 linker, but not the hinge domain
SEQ ID NO: 10 preferred nt sequence / PET1073G12
SEQ ID NO: 11 HCDR1 / PET1074B9
SEQ ID NO: 12 HCDR2 / PET1074B9
SEQ ID NO: 13 HCDR3 / PET1074B9
SEQ ID NO: 14 VCDR1 / PET1074B9
SEQ ID NO: 15 VCDR2 / PET1074B9
SEQ ID NO: 16 VCDR3/ PET1074B9
SEQ ID NO: 17 Heavy chain variable domain / PET1074B9
SEQ ID NO: 18 Light chain variable domain / PET1074B9
SEQ ID NO: 19 scFv / PET1074B9 including (G4S)3 linker, but not the hinge domain
SEQ ID NO: 20 preferred nt sequence / PET1074B9
SEQ ID NO: 21 HCDR1 / PET1287A10
SEQ ID NO: 22 HCDR2 / PET1287A10
SEQ ID NO: 23 HCDR3 / PET1287A10
SEQ ID NO: 24 VCDR1 / PET1287A10
SEQ ID NO: 25 VCDR2 / PET1287A10
SEQ ID NO: 26 VCDR3/ PET1287A10
SEQ ID NO: 27 Heavy chain variable domain / PET1287A10
SEQ ID NO: 28 Light chain variable domain / PET1287A10
SEQ ID NO: 29 scFv / PET1287A10 including (G4S)3 linker, but not the hinge domain
SEQ ID NO: 30 preferred nt sequence / PET1287A10
SEQ ID NO: 31 human IgG4 hinge domain
SEQ ID NO: 32 human CD8 hinge domain.
SEQ ID NO: 33 transmembrane domains of human CD8
SEQ ID NO: 34 transmembrane domains of human CD28
SEQ ID NO: 35 transmembrane domains of human CD137
SEQ ID NO: 36 intracellular signaling domain of human CD137
SEQ ID NO: 37 intracellular part of human CD8
SEQ ID NO: 38 FLAG tag
SEQ ID NO: 39 --
SEQ ID NO: 40 murine kappa light chain leader sequence
SEQ ID NO: 41 switch receptor (SR01)
SEQ ID NO: 42 preferred switch receptor (SR02)
SEQ ID NO: 43 switch receptor (SR03)
SEQ ID NO: 44 switch receptor (SR04)
SEQ ID NO: 45 switch receptor (SR05)
SEQ ID NO: 46 switch receptor (SR06)
SEQ ID NO: 47 switch receptor (SR07)
SEQ ID NO: 48 switch receptor (SR08)
SEQ ID NO: 49 switch receptor (SR09)
SEQ ID NO: 50 switch receptor (SR10)
SEQ ID NO: 51 switch receptor (SR11)
SEQ ID NO: 52 switch receptor (SR12)
SEQ ID NO: 53 switch receptor (SR1-2)
SEQ ID NO: 54 switch receptor (SR2-2)
SEQ ID NO: 55 switch receptor (SR3-2)
SEQ ID NO: 56 nucleic acid encoding SR02

### Examples

### MATERIALS & METHODS

For the generation of transduced T cells, PBMCs were isolated from fresh blood via gradient centrifugation and T cells contained therein were stimulated with bead-immobilized anti-CD3 and anti-CD28 antibodies (TransAct, Miltenyi GmbH) in medium supplemented with a T-cell stimulatory cytokine. For example, IL-2, IL-7 and/or IL-15 may be used. On days 2 and 3 after stimulation, cells were transduced with a retrovirus containing a transgene (e.g. SEQ ID NO 41-55). The retrovirus was generated by lipofection of GALV cells with plasmid DNA of the transgene(s) using Lipofectamine transfection kit (Invitrogen) according to the manufacturer's instructions. Transduced T cells were further cultivated for 9 to 12 days and used for assays. The expression of the SWITCHes was assessed by flow cytometry with antibodies against the FLAG tag.

### Determination of functional avidity

To determine the functional avidity, a total of 10⁴ T cells with SWITCH transduction efficiency of > 70 % (measured by flow cytometric detection of FLAG) were stimulated with a 1:300 dilution of aCD3/aCD28 beads (TransAct, Miltenyi GmbH) in the presence of human recombinant TGFβ1 (Peprotech) in a range of concentrations, for 24 hours. The assay was performed in a 96-well plate in a total volume of 200µl of serum-free culture medium. IFNy was detected in the supernatant (1:5 dilution) by ELISA (BD Biosciences). IFNy values were then normalized to % of maximum IFNy and plotted in a logarithmic concentration scale. A non-linear curve fit was performed in order to determine the EC₅₀ of the SWITCH receptor, i.e., the concentration of TGFβ in which the IFNγ release is 50 % of maximum.

### RESULTS

### Immunosuppressive effect of TGFβ on T cells

In order to demonstrate the suppressive effect of TGFβ on the function of T cells, the inventors designed an assay in which T cells are polyclonally restimulated via their TCR with beads coated with anti-CD3 and anti-CD28 antibodies (TransAct, Miltenyi GmbH) in the presence or absence of TGFβ. After 24 hours, the cytotoxicity mediator cytokine IFNy was detected in the supernatant as a surrogate of T cell function. Prior to stimulation, the T cells were expanded for nine days with a lab-scale manufacturing protocol; however, they were not engineered with tumor-specific TCRs at this point, but the T cells carried their natural TCRs.

In this assay (Fig. 1), the immunosuppressive effect of TGFβ on T cells was observed, as T cells activated in the presence of TGFβ secreted less IFNy (40% reduction) than those not exposed to TGFβ. This experimental setup is thus suitable to assess the capacity of SWITCH candidates to reverse the inhibitory effect of TGFβ.

### SWITCH construct design

The inventors' goal was to engineer an advantageous transmembrane SWITCH that binds extracellular TGFβ and translates the signal to the T cell, thereby reversing TGFβ's inhibitory effect. As an important safety feature, this costimulatory signal alone must not be sufficient to activate the T cells in the absence of a TCR signal. Thus, T cells carrying the SWITCH remain quiescent in the presence of TGFβ until they reach their target tumor cells. The inventors chose a costimulatory CD137 intracellular domain or a fusion between part of a CD8 intracellular domain and the CD137 intracellular domain for this end.

The SWITCH was designed as a modular construct containing multiple domains (Fig. 2) and 15 candidate constructs that differ in one or more domains were synthesized (Table 1). The TGFb binding domains are single-chain variable fragments (scFv) constructed based on three anti-TGFβ antibodies (PET1073G12 (scFv sequence SEQ ID NO: 9), PET1074B9 (scFv sequence SEQ ID NO: 19, and PET1287A10 (scFv sequence SEQ ID NO: 29) described in a patent (WO2006086469A2 (*23*)) by the Genzyme Corporation.

### Other published constructs

In a literature search, three other engineered receptors that bind to TGFβ and either provide a positive signal or no signal at all to the T cell were identified. These receptors all have different structures and domains than the present candidate SWITCHes. These receptors were used to benchmark the SWITCHes of the invention. BM (benchmarking receptor) 1 is a chimeric antigen receptor (CAR) against soluble TGFβ with an scFv binding domain derived from the PET1074B9 antibody and intracellular domains of CD28 and CD3ζ (*16*). BM2 is composed of the ectodomain of TGFβ receptor II (TGFβRII) and transmembrane and intracellular domains of CD137 (12). BM3 is a truncated version of TGFβRII, which binds soluble TGFβ but does not transmit any signal (17).

### SWITCH Candidate Selection

In order to assess expression levels and functionality, each of the present candidate SWITCHes and the three benchmarking receptors were cloned into a retroviral vector backbone and a retroviral transduction of activated human CD8+ T cells performed. The expression level of the SWITCHes was assessed by flow cytometry via staining of the FLAG tag (or TGFβRII for BM3) on the cell surface. Most SWITCH candidates, with the exception of SR1-2, SR2-2-, and SR3-2, had excellent transduction rates and surface expression levels (Fig. 3). Of the benchmarking receptors, BM1 and BM3 showed high expression while BM2 showed intermediate expression.

In order to assess the efficacy of the SWITCHes in reversing the inhibitory effect of TGFβ, the above-mentioned polyclonal activation assay was performed with αCD3/αCD28 beads in the absence or presence of TGFβ. TGFβ reduced the IFNy secretion by non-engineered T cells; cells expressing the SWITCHes were not only protected from this suppressive effect but secreted more IFNy when activated by αCD3/αCD28 in the presence of TGFβ (Fig. 4A). In order to normalize the data and compare the constructs, we calculated the fold-change in IFNy secretion of cells activated with αCD3/αCD28 + TGFβ versus αCD3/αCD28 alone for each construct (Fig. 4Fig. 1. IFNy release by T cells in the presence of TGFβ. T cells from 2 donors were expanded with a lab-scale protocol for 9 days prior to restimulation with aCD3/aCD28 beads (1:100 dilution) in the presence or absence of 10 ng/ml human recombinant TGFβ for 24 hours. IFNy in the supernatant was measured via ELISA. Graph shows mean±SEM IFNy concentration from replicates of 2 donors.

Fig. 2. Modular building blocks of switch receptors. Leader: murine kappa light. FLAG: tag for specific detection and/or selection of the SWITCH. TGFβ binding domain: VH and VL chains of one of three human anti-TGFβ antibodies. Hinge: human IgG4 or CD8. Transmembrane (TM) domain: human CD137, CD8, or CD28. Signaling domain: human CD137 or truncated human CD8 together with CD137.

Fig. 3. Cell surface expression of SWITCH and BM receptors. Human CD8+ T cells were retrovirally transduced with the 15 SWITCH candidates or with 3 BM receptors. Transduction efficiency and surface expression levels were assessed on day 5 after transduction by flow cytometric staining of the FLAG tag or TGFβRII (only for BM3). Dot plots show live T cells from 1 of 2 donors. Number represents frequency of cells expressing the engineered receptors.

Fig. 4B). TGFβ caused a 40 % reduction in IFNy secretion by non-engineered cells (fold change of 0.6). The BM1 and BM2 receptors were able to protect the T cells from this negative effect, leading to fold-changes of 3.4 and 1.2, respectively. BM3 cells did not fully circumvent TGFβ with a fold-change of 0.87. Our SWITCH receptors all showed a fold-change above 1, i.e., they converted the negative signal into a positive one and led to increased IFNy secretion. SR01-04 outperformed the other constructs, including the benchmarking receptors, with a fold-change between 3.5 and 5.

In order to further explore the differences between the candidate constructs and select a lead, the experiment was repeated with a 3-fold lower concentration of aCD3/aCD28 beads with the intention of providing sub-optimal TCR stimulation and thereby accentuate the contribution of the SWITCH signal to the T cell's activation. In this setting, the absolute IFNy secretion by non-engineered T cells was reduced by 40 % (Fig. 5A) compared to the stimulation with the optimal concentration of aCD3/aCD28 beads (Fig. 4A) and the suppressive effect of TGFβ was stronger (fold-change of 0.55). The BM1 and BM2 receptors were able to protect the T cells from this negative effect with a fold-change of 3.4 and 1.9, respectively. BM3 cells did not fully circumvent TGFβ with a fold-change of 0.7. All novel SWITCH receptors protected the T cells from TGFβ, but the differences between the best-performing constructs (SR01-SR04) became more apparent in this setting. SR02 and SR04 both showed the highest fold-change of about 6-fold (Fig. 5B). Importantly, those constructs outperformed all three benchmarking receptors.

To assess the safety of the SWITCH constructs, IFNy release in the absence of aCD3/aCD28 beads was also measured in order to assess whether the SWITCH is capable of stimulating the T cells on its own when in contact with TGFβ, a feature that would be undesirable. SWITCH-engineered T cells circulating the body on specific tissues must not be activated when encountering TGFβ. The SWITCH should only work in conjunction with the TCR to specifically boost the anti-tumor effect of the T cell.

Most SWITCHes did not activate the T cells, only SR1-2, SR01, SR04, and SR12 showed some minor levels of IFNy secretion in the presence of TGFβ alone (Fig. 6). Of note, BM1, which is a TGFβ-CAR that contains the two intracellular signalling domains of CD28 and CD3ζ activated the T cells in the presence of TGFβ alone, thus, the BM1 construct would not be suitable as a SWITCH in tumor-specific TCR- or CAR-T cells because T cells carrying BM1 may get activated at tissue sites outside of the tumor, which may cause toxicities.

In order to select a lead, a more thorough assessment of the two top candidates, SR02 and SR04 was performed. To that end, their function when activated (by aCD3/aCD28 beads) was evaluated in the presence of a range of TGFβ concentrations (0.4 - 50 ng/mL). Total IFNy secreted after overnight activation was measured and the fold-change in relation to activation without TGFβ calculated. SR02+ T cells secreted more IFNy than SR04+ T cells over a broad range of TGFβ concentrations (0.4 - 12.5 ng/mL) (Fig. 7A). The fold-change of SR02 was also higher at lower TGFβ concentrations between 0.4 and 6.2 ng/mL, a range that may be close to the physiological concentration of TGFβ (Fig. 7B). SR04+ T cells on the other hand showed a tendency to release higher IFNy at unphysiologically high concentrations of TGFβ.

In order to further assess the safety of the SWITCH receptors and whether SR02 or SR04 cause any background activation of T cells, IFNy secretion was measured over a range of TGFβ concentrations in the absence of TCR (CD3/CD28) stimulation. BM1 was also included in this experiment, as it can activate T cells in the absence of a TCR signal (Fig. 6). Both SR02- and SR04-expressing T cells did not secrete significant levels of IFNy in response to any of the different concentrations of TGFβ (Fig. 8). In contrast, BM1 showed a clear TGFβ dose-dependent IFNy secretion. TGFβ can be present in healthy tissues; therefore, activation of engineered cells by TGFβ alone could lead to unwanted toxicities. The SWITCH receptors of the invention do not activate T cells when a concomitant TCR stimulus is absent and thus have a more favourable safety profile than a classical BM1, which employs a classical CAR design.

Based on the assays performed, SR02 was chose as the preferred, lead candidate. SR02 showed excellent surface expression and effective rescue of T cells from the deleterious effects of TGFβ without any measurable background activation of the T cells by physiological concentrations of TGFβ. Of note, the scFv of SR02 is different than the one used by BM1. The modular composition of SR02 is shown in Fig. 9.

### Functional profiling of SWITCH (SR02) - Polyfunctionality

In the previous assays IFNy was measured as a surrogate of T cell activation and it was observed that cells expressing SR02 are resistant to the suppressive effect of TGFβ and even secrete higher amounts of IFNy when activated in the presence of TGFβ. Next, these findings were expanded to other cytokines important for T cell anti-tumor function. For this purpose, a multiplex cytometric bead array (CBA) covering 13 cytokines and cytotoxic mediators involved in CD8+ T cell function was used. SR02 supernatants from the cultures shown in Fig. 5 (aCD3/aCD28 beads 1:300 dilution) and Fig. 6 (no TCR stimulation) were measured with the CBA (LEGENDplex by Biolegend). In addition to IFNγ, IL-2 and TNFα were also secreted at higher amounts by SR02-expressing T cells when activated by aCD3/aCD28 in the presence of TGFβ (Fig. 10A). IL-2 is a potent stimulator of T cell function and survival, while TNFα is a pro-inflammatory cytokine with intrinsic tumoricidal activity. This indicates that the protective effect of SR02 against suppression by TGFβ is not limited to a single cytokine, but broadly boosts cytotoxic T cell function. Interestingly, the secretion of granzyme A and granzyme B (GrzA and GrzB, respectively) was not affected by TGFβ in this 24h assay. This might be due to the fact that these enzymes are already present in granules and are secreted right upon activation as opposed to IFNγ, IL-2 and TNFα which first need to be expressed and translated. The other cytokines measured by the CBA were only detected in low concentrations (IL-4, IL-10, IL-6, IL-17A, sFas, sFasL, Perforin and Gnly), and thus the effect of SR02 was not measurable. SR02-expressing T cells also did not show autonomous secretion of cytokines beyond basal levels when exposed to TGFβ in the absence of a TCR stimulus (Fig. 10B).

### Functional profiling of SWITCH-expressing T cells - Repeated stimulation

In addition to the short-term effect on cytokine secretion, TGFβ can reduce the long-term proliferation and survival of T cells. To address this problem, it was tested whether SR02 can protect T cells from this deleterious effect using a repeated stimulation assay, which simulates the conditions in the tumor microenvironment of constant antigen and TGFβ exposure. Non-engineered and SR02-expressing T cells were stimulated with aCD3/aCD28 beads every 2-3 days in the presence or absence of 10 ng/ml TGFβ, and cell numbers and viability were monitored between each stimulation (stimulations on days 0, 3, 5, 7, 10, 12 and 14). In the absence of TGFβ, both non-engineered and SR02+ T cells increased in numbers for 6 stimulations (d14) after which they started dropping (Fig. 11A). The viability of both cell populations remained stable for 4 stimulations (d10) and then slowly dropped until the end of the observation period (d17) (Fig. 11B). In the presence of TGFβ, non-engineered T cells expanded for only two stimulations (d5) and then quickly dropped in numbers and viability. By day 10 (after 4 stimulations), very few live T cells were left (~1.2 % viability) and the stimulations could not be continued (Fig. 11C & D). In contrast, SR02+ T cells expanded for 4 stimulations in the presence of TGFβ, after which they slowly dropped in numbers. Their viability started to slowly drop after the 3rd stimulation (day 7) and at the end of the experiment reached ~8 % (Fig.11 C & D).

This assay showed a clear advantage of SR02+ T cells over non-engineered T cells when exposed to harsh conditions of repeated stimulation and TGFβ exposure, as is the case in vivo in the tumor microenvironment. On d10, the SR02+ T cell number was 8.7-fold higher compared to the starting cell number, while non-engineered cells had already contracted and were 0.08-fold of the starting cell number, a difference of about 100-fold. This drastic improvement of T cell durability provided by SR02 can lead to profound improvements in clinical outcomes.

With regard to safety, this assay also showed that SR02+ T cells do not expand indefinitely when exposed to TGFβ and are thus very unlikely to develop into autonomously-dividing T cell leukemias and/or lymphomas.

### CONCLUSION

Clinical benefit to patients treated with receptor-engineered immune cell therapies might depend on the immune cells' ability to overcome the suppressive action of TGFβ in the TME of solid tumors. With this in mind, the inventors developed 15 switch receptor candidates to transform the negative signal from TGFβ into a positive one. They tested each candidate, together with 3 benchmarking receptors, in an assay designed to assess the capacity of the constructs to rescue T cell function from inhibition by TGFβ. Furthermore, they tested whether the SR candidates induced T cell activation when exposed to TGFβ in the absence of TCR stimulation. SR01-SR04 all significantly outperformed all state-of-the-art benchmarking receptors, as well as the other tested constructs.

SR02 was chosen as the lead SWITCH construct based on its expression level and capacity to efficiently convert TGFβ's negative signal into a stimulatory one in the absence of background activation. Further characterization of SR02+ T cells showed that they maintain their function in the presence of TGFβ and secrete high amounts of cytokines (IL-2, IFNy and TNFα) only when stimulated via their TCR. SR02+ T cells are significantly better suited than regular T cells to proliferate and survive in harsh conditions of repeated stimulations in the presence of TGFβ. Together, the improved function and increased durability of SR02+ T cells are decisive features that may contribute to better efficacy and improved outcomes for solid tumor patients.

The combination of the SWITCH receptors of the invention, in particular, SR02 with tumor-specific TCRs represents a next-generation approach with great potential for efficacious treatment of solid tumors with a highly suppressive tumor microenvironment.

### References

1. F. Cavallo, C. De Giovanni, P. Nanni, G. Forni, P.-L. Lollini, Cancer Immunol Immunother. 60, 319-26 (2011).
2. D. Hanahan, R. a Weinberg, Cell. 144, 646-74 (2011).
3. D. V. F. Tauriello, E. Sancho, E. Bathe, Not Rev Cancer. 22, 25-44 (2022).
4. S. F. Shariat et al., Clinical Cancer Research. 10, 1992-1999 (2004).
5. H. Tsushima et al., Gastroenterology. 110, 375-382 (1996).
6. S. Desruisseau et al., BrJ Cancer. 94, 239-246 (2006).
7. S. Mariathasan et al., Nature. 554, 544-548 (2018).
8. C. J. Martin et al., Sci Transl Med. 12, 1-16 (2020).
9. G. M. Bendle, C. Linnemann, L. Bies, J.-Y. Song, T. N. M. Schumacher, J Immunol. 191, 3232-9 (2013).
10. C. M. Bollard et al., Journal of Clinical Oncology. 36, 1128-1139 (2018).
11. S. Sukumaran et al., Cancer Discov. 8, 972-987 (2018).
12. T. L. Roth et al., Cell. 181, 728-744.e21 (2020).
13. WO2014172584A1.
14. WO2021244486 A1.
15. WO2017075433 A1.
16. Z. L. Chang et al., Not Chem Biol. 14, 317-324 (2018).
17. J. D. Silk et al., The Journal of Immunology. 208, 169-180 (2022).
18. EP3769816A1.
19. WO 2017/158019 A1.
20. WO 2009/003671 A2.
21. WO 2017/158029 A1.
22. WO 2022/243514 A1.
23. WO2006086469A2.

## Claims

1. A nucleic acid encoding a switch receptor comprising
a) a TGFβ - binding domain that is an scFv,
b) a hinge domain,
c) a transmembrane domain,
d) an intracellular signaling domain
an intracellular costimulatory signaling domain, wherein, after binding of the switch receptor to TGFβ, the intracellular costimulatory signaling domain is not capable of activating a T cell in the absence of further stimulation.
wherein, optionally, the intracellular signaling domain comprises an intracellular signaling domain of CD137, CD28, CD4 and/or CD8.

2. The nucleic acid of claim 1, wherein the intracellular signaling domain is the intracellular signaling domain of CD137 or a combination of an intracellular part of human CD8 and the intracellular signaling domain of human CD137.

3. The nucleic acid of any of the preceding claims, wherein the scFv comprises a variable heavy (VH) and a variable light (VL) domain, wherein
a) the VH domain comprises a CDR1 of SEQ ID NO: 1, a CDR2 of SEQ ID NO: 2 and a CDR3 of SEQ ID NO: 3 and the VL domain comprises a CDR1 of SEQ ID NO: 4, a CDR2 of SEQ ID NO: 5 and a CDR3 of SEQ ID NO: 6, or
b) the VH domain comprises a CDR1 of SEQ ID NO: 11, a CDR2 of SEQ ID NO: 12 and a CDR3 of SEQ ID NO: 13 and the VL domain comprises a CDR1 of SEQ ID NO: 14, a CDR2 of SEQ ID NO: 15 and a CDR3 of SEQ ID NO: 16, or
c) the VH domain comprises a CDR1 of SEQ ID NO: 21, a CDR2 of SEQ ID NO: 22 and a CDR3 of SEQ ID NO: 23 and the VL domain comprises a CDR1 of SEQ ID NO: 24, a CDR2 of SEQ ID NO: 25 and a CDR3 of SEQ ID NO: 26,
preferably, a).

4. The nucleic acid of claim 3, wherein the scFv comprises
a) a variable heavy (VH) domain of SEQ ID NO: 7 and a variable light (VL) domain of SEQ ID NO: 8, wherein the scFv optionally has SEQ ID NO: 9, or
b) a variable heavy (VH) domain of SEQ ID NO: 17 and a variable light (VL) domain of SEQ ID NO: 18, wherein the scFv optionally has SEQ ID NO: 19, or
c) a variable heavy (VH) domain of SEQ ID NO: 27 and a variable light (VL) domain of SEQ ID NO: 28, wherein the scFv optionally has SEQ ID NO: 29,
preferably, a).

5. The nucleic acid of claim 4, wherein the scFv is PET1073G12 of SEQ ID NO: 9 or a variant thereof having at least 90% sequence identity to SEQ ID NO: 9, preferably, PET1073G12 of SEQ ID NO: 9.

6. The nucleic acid of any of the preceding claims, wherein the hinge domain is an IgG hinge domain, e.g., an IgG4 hinge domain, a CD28 hinge domain or a CD8 hinge domain, preferably, an IgG4 hinge domain.

7. The nucleic acid of any of the preceding claims, wherein the transmembrane domain is selected from the group comprising transmembrane domains of CD8, CD28 or CD137, preferably, the transmembrane domain of CD8.

8. The nucleic acid of any of the preceding claims, wherein the switch receptor comprises the intracellular signaling domain of CD137.

9. The nucleic acid of any of the preceding claims, wherein the switch receptor comprises a combination of an intracellular part of human CD8 and the intracellular signaling domain of human CD137.

10. The nucleic acid of any of the preceding claims, wherein the switch receptor further comprises an extracellular tag, optionally, a FLAG tag N-terminal to the TGFβ-binding domain.

11. The nucleic acid of any of the preceding claims, wherein the switch receptor further comprises an N-terminal leader sequence, optionally, a murine kappa light chain leader sequence.

12. The nucleic acid of any of the preceding claims, wherein the switch receptor has the sequence of any of SEQ ID NO: 41-55 or at least 90% sequence identity to any of SEQ ID NO: 41-55, preferably, SEQ ID NO: 42.

13. A switch receptor encoded by the nucleic acid of any of claims 1-12.

14. A cell comprising the nucleic acid of any of claims 1-12,
wherein the cell optionally is a human T cell, NK cell or NKT cell, e.g., a T cell,
wherein the cell preferably expresses the switch receptor.

15. A pharmaceutical composition comprising the nucleic acid of any of claims 1-12, the switch receptor of claim 13 and/or the cell of claim 14 that is a human T cell, NK cell or NKT cell,
wherein the pharmaceutical composition optionally further comprises a pharmaceutically acceptable buffer or excipient.

16. The pharmaceutical composition of claim 15 for use in treatment of cancer and infectious diseases,
optionally, for use in adoptive T cell therapy.
